# FASCICULE DE BREVET EUROPEEN

(11) **EP 1 159 968 B1**
(45) Date de publication et mention de la délivrance du brevet: **29.10.2008**
(21) Numéro de dépôt: 00420113.3
(22) Date de dépôt: 30.05.2000
(51) Int. Cl.: C12N 7/04, A61K 39/12

(54) **Souches atténuées du virus de la Dengue et leur utilisation dans une composition vaccinale**
Attenuierte Stammen von Denguevirus und ihre Verwendungen in Impfstoffzusammensetzungen
Attenuated strains of Dengue virus and their use in a vaccine composition

(43) Date de publication de la demande: 05.12.2001
(73) Titulaire: Mahidol University, Bang Yi Khan - Bangkok-Noi, Bangkok 10700 (TH)
(72) Inventeur: Bhamarapravat, Nath Vaccine Development Center, Nakhonpathon 73170 (TH); Yoksan, Sutee Vaccibe Development Center, Nakhonpathon 73170 (TH)
(74) Mandataire: Kerneis, Daniéle

(56) Documents cités:
- WO-A-96/40933
- N. BHAMARAPRAVATI ET AL.: "Live attenuated tetravalent dengue vaccine" VACCINE, vol. 18, 2000, pages 44-47, XP002160013
- M. KHIN ET AL.: "Infection, dissemniation, transmission and biological attributes of dengue-2 PDK53 candidate vaccine virus after oral infection in aedes aegyptii" AM. J. TROP. MED. HYG., vol. 51, 1994, pages 864-869, XP000982036
- N. BHAMARAPRAVATI ET AL.: "Immunization with e live attenuated dengue-2 virus candidate vaccine (16681-PDK 53" BULLETIN OF THE WHO, vol. 65, no. 2, 1987, pages 189-195, XP000982026
- N. JIRAKANJANAKIT ET AL.: "Dynamics of susceptibility and transmissibility of the live attenuated candidate vaccines dengue-1 PDK13, dengue-3 PGMK30F3, and dengue-4 PDK48 after oral infection in aedes aegypti" AM. J. TROP. MED. HYG., vol. 61, no. 4, 1999, pages 672-676, XP000982031
- N. JIRAKANJANAKIT ET AL.: "The use of Toxorhynchites splendens for identification and quantitation of serotypes contrained in the tetravalent live attenuated dengue vaccine" VACCINE, vol. 17, 1999, pages 597-601, XP002160014
- D. VAUGHN ET AL.: "Testing of a dengue 2 live attenuated vaccine (strain 16681 PDK 53) in ten American volunteers" VACCINE , vol. 14, no. 4, - 1996 pages 329-336, XP002160015

## Description

L'invention est relative au domaine des virus de la Dengue et plus particulièrement à des souches atténuées du virus de la Dengue, ainsi qu'à une composition vaccinale comprenant au moins une de ces souches.

La Dengue, ainsi que sa forme sévère, la fièvre hémorragique est une maladie tropicale d'origine virale. Le virus qui en est responsable fait partie de la famille des Flaviviridae (genre Flavivirus), qui comprend également le virus de la fièvre jaune ainsi que celui de l'encéphalite japonaise. On connaît 4 sérotypes susceptibles de provoquer la Dengue. Alors que cette maladie est actuellement en progression dans le monde, aucun vaccin n'est actuellement disponible commercialement. En effet, pour pouvoir être proposé avec toutes chances de prévenir efficacement la Dengue, un vaccin doit, tout en présentant des garanties d'inocuité, être capable d'induire chez l'individu auquel il est administré une réponse du système immunitaire susceptible de le protéger lors d'une contamination ultérieure par les virus sauvages. Or, jusqu'à présent, aucun des candidats-vaccins étudiés n'a présenté toutes les qualités nécessaires.

Il existe donc un besoin de disposer de souches de virus de la Dengue suffisamment atténuées pour pouvoir être administrées en toute sécurité à des êtres humains, et notamment à des enfants, tout en ayant conservé un pouvoir d'infection et un caractère immunogène suffisants pour induire une réponse immunitaire permettant une prévention de la maladie.

Afin d'atteindre ce but, l'invention propose une souche isolée atténuée du sérotype 1 du virus de la Dengue, désignée sous la référence 16007 PDK 13 et déposée à la CNCM de l'Institut Pasteur de Paris sous la référence I-2480.

L'invention a également pour objet une souche isolée atténuée du sérotype 2 du virus de la Dengue, désignée sous la référence 16881 PDK 53 et déposée à la CNCM de l'Institut Pasteur de Paris sous la référence I-2481.

L'invention a également pour objet une souche isolée atténuée du sérotype 3 du virus de la Dengue, désignée sous la référence 16562 PGMK 30- FrhL 3 et déposée à la CNCM de l'Institut Pasteur de Paris sous la référence I-2482.

L'invention a encore pour objet une souche isolée atténuée du sérotype 4 du virus de la Dengue, désignée sous la référence 1036 PDK 48 et déposée à la CNCM de l'Institut Pasteur de Paris sous la référence I-2483.

L'invention a également pour objet une composition vaccinale comprenant au moins une souche atténuée du virus de la Dengue choisie parmi les souches suivantes: la souche I-2480, la souche I-2481, la souche I-2482, et la souche I-2483, ou une souche qui est dérivée de l'une d'entre elles.

L'invention a également pour objet l'utilisation d'une souche atténuée du virus de la Dengue choisie parmi les souches suivantes: la souche I-2480, la souche I-2481, la souche I-2482, et la souche I-2483, ou de toute souche qui en est dérivée pour la fabrication d'une composition vaccinale.

L'invention a également pour objet une méthode d'immunisation contre la Dengue, selon laquelle on administre à des adultes ou des enfants, une composition vaccinale comprenant au moins une souche atténuée du virus de la Dengue choisie parmi les souches suivantes: la souche I-2480, la souche I-2481, la souche I-2482, et la souche I-2483, ou une souche qui en est dérivée.

Les souches de virus selon la présente invention ont toutes été obtenues à partir de sérum de patients ayant été atteints de la Dengue en Asie du Sud-Est. Les isolements ont été passés sur cellules de mammifères puis inoculés par voie intra-thoracique (IT) à des moustiques d'élevage *(Toxorhynchites* ou *Aedes aegypti*). Cette étape a permis d'éliminer d'éventuels virus contaminants qui ne seraient pas des arbovirus ; en effet, seuls les arbovirus migrent au niveau des glandes salivaires du moustique après une administration intra-thoracique ; il fallait donc séparer la tête du reste du corps du moustique et la broyer pour obtenir la souche virale purifiée.
Ensuite, les virus des sérotypes 1, 2 et 4 ont été passés sur des cellules primaires de rein de chien (PDK), à 32° C, tous les 5 à 10 jours, alors que le virus du sérotype 3 a été atténué par passage sur des cellules de rein de singe (PGMK) puis cultivé sur des cellules pulmonaires de foetus Rhesus (FRhL).
Les souches atténuées que l'on obtient ont alors les caractéristiques suivantes:
- Lorsqu'on effectue des titrages sur cellules de mammifères, par exemple sur cellules LLC-MK2 qui sont des cellules issues de rein de singe, les souches atténuées donnent de petites plages comparativement aux souches parentales dont elles dérivent.
- Chez le souriceau nouveau-né, lorsqu'elles sont inoculées par voie intra-cérébrale, elles ont une faible virulence.
- Chez le singe, lorsqu'elles sont inoculées par voie sous-cutanée, elles provoquent une virémie faible (voire non-détectable).
- Elles ont une sensibilité à une température proche de 40°C.

Aux termes de la présente invention, on entend par souche dérivée des souches faisant l'objet des dépôts à la CNCM I-2480, I-2481, I-2482 et I-2483, des souches virales obtenues à partir de ces souches, mais qui ont subi des passages supplémentaires sur cellules permissives aux virus, tout en conservant les propriétés mentionnées ci-dessus.

La souche 16007, de sérotype 1, a été isolée en 1964 d'un patient atteint de Dengue associée à un syndrome de choc en Thailande. Le sérum virémique original a été passé sur des cultures de tissu avant d'être inoculé au moustique *Toxorhynchites amboinensis.*
Ensuite, le virus obtenu a été passé sur cellules PGMK pour préparer la semence virale-mère. La semence est ensuite passée sur cellules PDK, jusqu'à 43 fois. La souche obtenue après 13 passages sur cellules PDK présente toutes les caractéristiques d'atténuation pour une utilisation vaccinale ; c'est celle faisant l'objet du dépôt I-2480 effectué auprès de la CNCM.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2480 est considérée comme faisant partie de la présente invention.

La souche 16681, de sérotype 2, a été isolée d'un patient représentant un cas fatal de Dengue en Thailande en 1964. Cette souche a été passée 2 fois dans des moustiques *Toxorhynchites amboinensis* et une fois sur cellules de rein de singe pour produire la semence-mère. La souche 16681 PDK 53 convenant aux fins de l'invention et objet du dépôt I-2481 effectué auprès de la CNCM a été obtenue après 53 passages successifs hebdomadaires sur des cellules PDK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2481 est considérée comme faisant partie de la présente invention.

La souche 16562, de sérotype 3, a été isolée d'un patient atteint de Dengue à Manille en 1972. Elle a atténuée par 30 passages successifs sur cellules PGMK, puis cultivée sur cellules FRhL. La souche vaccinale 16562 PGMK 30 FRhL 3, objet du dépôt I-2482 effectué auprès de la CNCM et convenant aux fins de l'invention a subi 3 passages successifs sur cellules FRhL après les 30 passages sur cellules PGMK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PGMK ou FRhL, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2482 est considérée comme faisant partie de la présente invention.

La souche 1036, de sérotype 4, a été isolée d'un enfant atteint de Dengue classique en Indonésie. Cette souche a été atténuée par passages successifs sur des cellules PDK. La souche 1036 PDK 48, convenant aux fins de l'invention et objet du dépôt I-2483 effectué auprès de la CNCM a été passé 48 fois sur cellules PDK.
Bien que cette souche soit celle préférée pour la composition vaccinale selon l'invention, il est tout à fait possible d'utiliser une souche qui en est dérivée, et notamment une souche ayant subi quelques passages supplémentaires sur cellules PDK, ou une souche ayant été cultivée ultérieurement sur des cellules lui permettant de conserver son caractère immunogène tout en n'accroissant pas son caractère virulent. Ainsi, des souches ayant été cultivées sur cellules VERO pourraient également convenir. Toute souche dérivée de la souche I-2483 est considérée comme faisant partie de la présente invention.

Les souches selon l'invention sont utilisées pour la préparation de compositions vaccinales. Ces compositions vaccinales peuvent être des compositions monovalentes, bivalentes, trivalentes ou tétravalentes en ce sens qu'elles peuvent comprendre de 1 à 4 sérotypes du virus de la Dengue. Elles peuvent également comprendre d'autres antigènes vaccinaux, ce qui permet alors de vacciner simultanément contre plusieurs maladies.

Dans chacun des cas, la quantité de virus de chacun des sérotypes est déterminée de manière que la dose administrée induise une production d'anticorps neutralisants vis-à-vis du sérotype administré, sans conduire cependant à des réactions secondaires indésirables. De bons résultats ont été obtenus grâce à des vaccins monovalents contenant 10⁵ pfu/ml lorsqu'il s'agissait du sérotype 4 et 10³ à 10⁴ pfu/ml lorsqu'il s'agissait des autres sérotypes.

Des essais ont également été réalisés avec des vaccins bivalents obtenus par simple mélange de 0,5 ml de chacun des sérotypes 1 et 2, ou bien 1 et 4, ou bien 2 et 4. Ainsi que cela a été décrit dans l'article de Bhamarapravati and Yoksan (1989) *« Study of bivalent vaccine in volunteers »,* Lancet, 1077, toutes les compositions vaccinales se sont révélées immunogènes et sans danger, conduisant à un taux de séroconversion de 100% pour chacune des formulations testées.

De la même façon, un vaccin trivalent obtenu par mélange de 0,33ml de chacun des sérotypes 1,2 et 4 a également été testé chez des patients et a conduit à un taux de séroconversion de 100% ainsi que cela a été décrit par Natth Bhamarapravati et SuteeYoksan dans « The clinical trial of trivalent Dengue vaccine », 1990, Southeast Asian Journal of Tropical Medicine and Public Health, Vol 21 (4) p.709.

On a également préparé une formulation tétravalente par mélange des formulations monovalentes, et obtenu une composition qui a conduit à une séroconversion de 100% des sujets vaccinés.

Selon un mode particulier de l'invention, la composition vaccinale peut également être administrée en 2 doses, qui peuvent être, soit des doses complémentaires si l'on considère les sérotypes qu'elles contiennent, soit des doses identiques destinées à être administrées 2 fois.

L'inoculation d'un vaccin monovalent ou contenant plusieurs sérotypes de Dengue à des volontaires préalablement immuns contre la Dengue ne s'est accompagnée d'aucune anomalie et notamment d'effets secondaires indésirables comme cela aurait pu être envisagé quand on se réfère à l'hypothèse des anticorps facilitants. Cette hypothèse élaborée par Halstead dans les années 60 précise qu'un facteur de risque de la Dengue hémorragique était la survenue d'une Dengue secondaire chez des sujets présentant des anticorps facilitants obtenus lors d'une première infection. En d'autres termes, l'apparition de la Dengue hémorragique semble être due à 2 infections successives par 2 sérotypes différents du virus de la Dengue. Dans les investigations conduites avec le vaccin un certain nombre de volontaires étaient préimmuns vis-à-vis d'un virus de la Dengue. Après vaccination il n'y a pas eu d'effets secondaires supérieurs en fréquence et en intensité comparativement aux sujets séro négatifs.

De plus, on a constaté qu'après l'administration de la 1^{ère} dose d'une composition selon l'invention, l'administration d'une 2^{nde} dose était parfaitement bien tolérée, et conduisait chez les sujets qui n'auraient pas eu une réponse vis-à-vis des 4 sérotypes à une complémentation de la réponse immunitaire, i.e. à une réponse en anticorps contre les sérotypes vis-à-vis desquels la réponse était négative lors de la 1^{ère} dose.

Les exemples suivants illustrent quelques modes particuliers de réalisation de la présente invention.

### Exemple 1: Essai clinique sur 10 volontaires avec un vaccin monovalent contre le sérotype 2 de la Dengue.

On dispose de la souche 16681 PDK 53 obtenue par passages successifs sur des cellules PDK, et préparée en albumine humaine à une concentration de 3,5%. La suspension virale est centrifugée à 1050 g pendant 1 heure à 0-4 °C. Le surnageant est filtré au moyen de filtres 0,22µm et réparti dans des ampoules de 1 ml qui sont stockées à ―80°C. Le titre viral du candidat vaccin va de 2 à 5x10⁴ pfu/ml sur cellules LLC-MK2. Les tests de contrôle de qualité ont été réalisés en accord avec les monographies applicables pour des vaccins viraux vivants atténués, et ont été trouvés satisfaisants.
Les doses destinées à être administrées en sous-cutané, sont préparées par mélange de 2 doses de 1ml, la moitié étant pour l'immunisation, l'autre moitié pour la détermination du titre viral.

La population objet de l'essai est constituée par 10 adultes de sexe masculin, habitant la province de Lampoon en Thailande. Leur âge est compris entre 18 et 30 ans. Les examens cliniques, biochimiques et hématologiques permettent de conclure à un bon état de santé, à l'exception de quelques cas présentant une infestation de parasites intestinaux avec une éosinophilie faible à modérée. Sur les 10 volontaires, 5 ne présentent aucun signe sérologique d'une précédente infection par la Dengue ou l'Encéphalite japonaise, lorsque les tests réalisés sont des tests de neutralisation et de réduction de plages (PRNT pour Plaque Reduction Neutralization Tests) ou des tests d'inhibition de l'hémagglutination (HI pour Haemagglutination Inhibition). Les 5 autres volontaires ont montré des réponses PRNT positives au virus de l'encéphalite japonaise (JEV pour Japanese Encephalitis Virus).
Après administration du candidat-vaccin, les volontaires sont examinés régulièrement pendant 21 jours.
On effectue des prises de sang aux jours suivants: J0, J6, J10, J14, J18, J21, J30, J60, J180, 1 an puis 1 an 1/2 après l'immunisation. Sur les échantillons sanguins recueillis, on effectue des tests d'isolement de virus, des dosages sérologiques, ainsi que des analyses biochimiques et hématologiques.

Les résultats de l'essai sont les suivants.
Aucun des volontaires n'a présenté de réactions secondaires graves, telles qu'une température élevée, des saignements, de l'hypotension, ou autre. Des réactions légères, telles que des maux de tête et des douleurs abdominales ont été notées chez la moitié des volontaires.
En ce qui concerne les analyses chimiques, aucune anomalie n'a été constatée durant l'étude.
Les analyses hématologiques ont montré que le nombre total de globules blancs a diminué chez tous les volontaires aux environs de J10 pour retourner vers des valeurs normales à J14 ou plus tard. Il n'y a pas eu de véritable leucopénie cependant. Au même moment, on a pu observer une lymphocytose, une monocytose et une formation de lymphocytes atypiques. D'autre part, chez 6 des 10 volontaires, on a observé la présence de plaquettes géantes sans thrombocytopénie.

Le titrage du vaccin inoculé a montré que les volontaires ont reçu entre 1,9 et 2,7 10⁴ pfu/ml. Une virémie a été constatée chez un des volontaires à J10, après amplification sur LLC-MK2 et C6/36. Le virus détecté formait de petites plages et ne poussait pas à 37,9 °C.

Les dosages immunologiques, et notamment les tests PRNT montrent que tous les volontaires ont développé une réponse en anticorps contre le sérotype 2 de la Dengue.
Les résultats sont présentés dans le tableau 5 ci-après.
Bien que cela n'apparaisse pas sur ce tableau, on a remarqué que certains volontaires ont également développé une réponse contre d'autres sérotypes de la Dengue; chez les volontaires qui présentaient, avant l'essai, des anticorps contre l'encéphalite japonaise, une réponse contre les 4 sérotypes de la Dengue a été observée, la réponse contre le sérotype 2 étant cependant plus importante.

**Tableau 5**

| Numéro du Volontaire | Titre en anticorps par PRNT après immunisation | | | | | | | |
|---|---|---|---|---|---|---|---|---|
| | 0 | J14 | J21 | J30 | J60 | J180 | 1 an | 1 an1/2 |
| 1 | <10 | <10 | 80 | 260 | 163 | 93 | 81 | 63 |
| 2 | <10 | 42 | 59 | 40 | 42 | 90 | 84 | 45 |
| 4 | <10 | 25 | 620 | 600 | 430 | 180 | 82 | 110 |
| 5 | <10 | 38 | 58 | 305 | 85 | 44 | 42 | 25 |
| 10 | <10 | <10 | 170 | 680 | 370 | 255 | 240 | 122 |
| 3 | <10 | 83 | 480 | 620 | 275 | 178 | 175 | 100 |
| 6 | <10 | 26 | 1160 | 1200 | 739 | 139 | 120 | 58 |
| 7 | <10 | 42 | 520 | 670 | 277 | 210 | 72 | 74 |
| 8 | <10 | 88 | 930 | 1150 | 460 | 169 | 180 | 75 |
| 9 | <10 | 15 | 200 | 400 | 89 | 75 | 110 | 37 |

Les volontaires 1, 2, 4, 5 et 10 (5 premières lignes) sont les 5 volontaires ne présentant pas d'anticorps contre l'encéphalite japonaise avant l'inoculation du présent essai, alors que les volontaires 3, 6, 7, 8 et 9 sont ceux qui ne présentaient pas d'anticorps contre la Dengue mais qui possédaient des anticorps contre l'encéphalite japonaise.

Ce tableau met en évidence le fait que le taux d'anticorps neutralisant est à son maximum autour de J21-J30 mais que ces anticorps persistent au cours du temps.

### Exemple 2: Essai clinique avec un vaccin monovalent contre le sérotype 1 de la Dengue

On dispose de la souche 16007, isolée d'un patient, à laquelle on fait subir un certain nombre de passages sur cellules PDK. On obtient donc des souches correspondant à différents niveaux d'atténuation, que l'on administre en sous-cutané à des volontaires Thailandais, sous forme d'une dose de 1ml.
Les résultats obtenus en terme de séroconversion 6 mois après la date de l'inoculation sont récapitulés dans le Tableau 6 ci-après:

Dans ce test, on considère qu'il y a séroconversion lorsqu'il apparaît des anticorps neutralisants à un titre supérieur à 1/10.

| Nombre de Passages sur cellules PDK | Taux de séroconversion (Nbre séroconversion/Nbre de volontaires) |
|---|---|
| PDK 43 | 17 ( 2/12) |
| PDK 30 | 40 (2/5) |
| PDK 20 | 60 (3/5) |
| PDK 13 | 78 ( 7/9) |

Ces résultats montrent que le taux de séroconversion est relativement faible lorsque les souches sont passées de 20 à 43 fois sur cellules PDK. Par contre, leur réactogénicité était également faible, ce qui est favorable. La souche étant trop atténuée, elle a en partie perdu son pouvoir immunogène.
Après 13 passages, ce qui correspond à la souche I-2480 objet du dépôt effectué à la CNCM, le candidat-vaccin présente toujours une réactogénicité minimale, mais par contre le taux de séroconversion après administration d'une seule dose est de 78 %.

Une seconde dose est administrée 1 mois après la 1^{ère} aux 2 volontaires n'ayant pas séroconverti avec la souche 16007 PDK 13; après cette 2^{nde} dose, le taux de séroconversion est alors de 100 %.

### Exemple 3: Essai clinique avec un vaccin monovalent contre le sérotype 3 de la Dengue.

On dispose de la souche 16562 ayant été atténuée par 30 passages sur cellules PGMK puis cultivée par 3 passages sur cellules FRhL; cette souche fait l'objet du dépôt effectué à la CNCM sous le N° I-2482.
Administrée à 4 volontaires, elle conduit à un épisode de fièvre de 38,5 °C chez l'un d'entre eux. Parmi les volontaires immunisés, 3 ont séroconverti dès la 1^{ère} dose; le volontaire n'ayant pas séroconverti a reçu une seconde dose 1 mois après la 1^{ère}, et a alors séroconverti également.

### Exemple 4: Essai clinique avec un vaccin monovalent contre le sérotype 4 de la Dengue.

On dispose de la souche 1036 qui a été atténuée par 48 passages successifs sur cellules PDK. Cette souche fait l'objet du dépôt CNCM N° I-2483 .Formulée sous forme vaccinale, elle est administrée à 5 volontaires et montre une réactogénicité minimale pour un taux de séroconversion de 100 %.

### Exemple 5 : Essai clinique avec des vaccins bivalents.

A partir des vaccins monovalents tels que ceux décrits aux exemples précédents, on prépare des vaccins bivalents. On prépare ainsi des doses de 1 ml par mélange de 0,5 ml de chacun des vaccins monovalents 1 et 2, 1 et 4, ainsi que 2 et 4. Les vaccins obtenus sont administrés à des volontaires de la même façon que les vaccins monovalents. Les résultats obtenus sont particulièrement satisfaisants, car les formulations vaccinales sont sûres et conduisent à une séroconversion de 100 % des sujets immunisés dès la 1^{ère} injection.

### Exemple 6: Essai clinique avec un vaccin trivalent.

A partir des vaccins monovalents des exemples 1, 2 et 4, on prépare un vaccin trivalent, sous forme de doses de 1 ml obtenue par mélange de 0,33 ml des vaccins monovalents des exemples précédents. Administré à des volontaires mâles de 19 à 35 ans, ce vaccin trivalent conduit à une séroconversion de tous les sujets immunisés dès la 1^{ère} injection.

### Exemple 7: Essai clinique avec un vaccin tétravalent chez une population d'adultes.

On prépare un vaccin tétravalent à partir des compositions monovalentes des exemples 1 à 4 précédents.

La composition de la dose vaccinale est indiquée dans le tableau 7 ci-après:

| Sérotype | Volume | Fourchette de concentration en virus (titre moyen) * |
|---|---|---|
| Dengue 1 | 0,4ml | 3000-5000 (4000) |
| Dengue 2 | 0,1ml | 800-1200 (1000) |
| Dengue 3 | 0,4ml | 3000-5000 (4000) |
| Dengue 4 | 0,1ml | 3000-5000(4000) |

| | | |
|---|---|---|
| *Résultats exprimés en pfu/ml. | | |

Cette composition a été administrée à 14 volontaires, parmi lesquels certains avaient un statut immunologique naïf vis-à-vis des Flavivirus alors que d'autres présentaient déjà des anticorps anti-Flavivirus avant l'administration du candidat-vaccin. Cependant, quel que soit leur statut immunologique préalable, tous les volontaires ont séroconverti lors de l'administration d'une seule dose du candidat-vaccin selon l'invention.
Au cours de cet essai, quelques réactions secondaires légères ont été observées chez les sujets vaccinés.

### Exemple 8 : Essai clinique avec un vaccin tétravalent chez une population d'enfants.

On dispose d'une composition vaccinale tétravalente. La population objet de l'essai est une population d'enfants ayant de 1 à 14 ans ; elle est segmentée de la façon suivante : les enfants entre 10 et 14 ans, les enfants entre 5 et 9 ans, et les enfants entre 1 an et 4 ans. On utilise différentes doses et différentes formulations. Environ 169 enfants sont immunisés. Dans le groupe des 5 à 9 ans, avec des doses allant de 4000 à 6000 pfu, on obtient un taux de séroconversion de presque 80 %. Des réactions cliniques légères consistant en de la fièvre (38°C) et un rash pendant 1 à 2 jours sont observées chez environ 1 % des immunisés.
A 22 enfants ayant reçu une 1^{ère} dose à 1350 pfu ml, on administre après 6 mois une 2^{nde} dose allant de 1300 à 3000 pfu/ml, et on obtient alors une forte réponse immunitaire. On observe alors, chez 20 sujets la présence d'anticorps neutralisants contre les 4 sérotypes. Ces résultats confirment l'intérêt de l'administration d'une seconde dose.

## Revendications

1. Souche isolée atténuée du sérotype 1 du virus de la Dengue, désignée sous la référence 16007 PDK 13 et déposée à la CNCM sous la référence I-2480.

2. Souche isolée atténuée du sérotype 2 du virus de la Dengue, désignée sous la référence 16881 PDK 53 et déposée à la CNCM sous la référence I-2481.

3. Souche isolée atténuée du sérotype 3 du virus de la Dengue, désignée sous la référence 16562 PGMK 30- FrhL 3 et déposée à la CNCM sous la référence I-2482.

4. Souche isolée atténuée du sérotype 4 du virus de la Dengue, désignée sous la référence 1036 PDK 48 et déposée à la CNCM sous la référence I-2483.

5. Composition vaccinale comprenant au moins une souche atténuée du virus de la Dengue selon une des revendications précédentes.

6. Composition vaccinale comprenant au moins une souche atténuée du virus de la Dengue dérivée d'une souche selon une des revendications 1 à 4.

7. Composition vaccinale comprenant au moins une souche de chacun des sérotypes 1 à 4 du virus de la Dengue, les souches étant les souches atténuées selon les revendications 1 à 4 ou des souches qui en sont dérivées.

8. Composition vaccinale selon une des revendications 5 à 7, **caractérisée en ce qu'**elle est destinée à être administrée en 2 doses.

9. Composition vaccinale selon une des revendications 5 à 7, **caractérisée en ce qu'**elle est destinée à être administrée par injection sous-cutanée, en 2 doses séparées par un intervalle de temps d'au moins 1 mois.

10. Utilisation d'au moins une des souches selon l'une des revendications 1 à 4 pour la fabrication d'une composition vaccinale contre la Dengue.

## Claims

1. Attenuated isolated strain of serotype 1 of the Dengue virus, designated by the reference 16007 PDK 13 and deposited at the CNCM under the reference I-2480.

2. Attenuated isolated strain of serotype 2 of the Dengue virus, designated by the reference 16881 PDK 53 and deposited at the CNCM under the reference I-2481.

3. Attenuated isolated strain of serotype 3 of the Dengue virus, designated by the reference 16562 PGMK 30- FrhL 3 and deposited at the CNCM under the reference I-2482.

4. Attenuated isolated strain of serotype 4 of the Dengue virus, designated by the reference 1036 PDK 48 and deposited at the CNCM under the reference I-2483.

5. Vaccine composition comprising at least one attenuated strain of the Dengue virus according to one of the preceding claims.

6. Vaccine composition comprising at least one attenuated strain of the Dengue virus derived from a strain according to one of Claims 1 to 4.

7. Vaccine composition comprising at least one strain of each of serotypes 1 to 4 of the Dengue virus, the strains being the attenuated strains according to Claims 1 to 4 or strains derived therefrom.

8. Vaccine composition according to one of Claims 5 to 7, **characterized in that** it is intended to be administered in 2 doses.

9. Vaccine composition according to one of Claims 5 to 7, **characterized in that** it is intended to be administered by subcutaneous injection, in 2 doses separated by a time interval of at least 1 month.

10. Use of at least one of the strains according to one of Claims 1 to 4 for the manufacture of a vaccine composition against Dengue.

## Patentansprüche

1. Isolierter attenuierter Stamm des Serotyps 1 des Dengue-Virus, der unter der Bezeichnung 16007 PDK 13 geführt wird und unter der Nummer I-2480 bei der CNCM hinterlegt ist.

2. Isolierter attenuierter Stamm des Serotyps 2 des Dengue-Virus, der unter der Bezeichnung 16881 PDK 53 geführt wird und unter der Nummer I-2481 bei der CNCM hinterlegt ist.

3. Isolierter attenuierter Stamm des Serotyps 3 des Dengue-Virus, der unter der Bezeichnung 16562 PGMK 30-FrhL 3 geführt wird und unter der Nummer I-2482 bei der CNCM hinterlegt ist.

4. Isolierter attenuierter Stamm des Serotyps 4 des Dengue-Virus, der unter der Bezeichnung 1036 PDK 48 geführt wird und unter der Nummer I-2483 bei der CNCM hinterlegt ist.

5. Impfstoffzusammensetzung, die mindestens einen attenuierten Stamm des Dengue-Virus nach einem der vorhergehenden Ansprüche enthält.

6. Impfstoffzusammensetzung, die mindestens einen von einem Stamm nach einem der Ansprüche 1 bis 4 abgeleiteten attenuierten Stamm des Dengue-Virus enthält.

7. Impfstoffzusammensetzung, die mindestens einen Stamm jedes der Serotypen 1 bis 4 des Dengue-Virus enthält, wobei es sich bei diesen Stämmen um attenuierte Stämme nach den Ansprüchen 1 bis 4 oder um von diesen abgeleitete Stämme handelt.

8. Impfstoffzusammensetzung nach einem der Ansprüche 5 bis 7, **dadurch gekennzeichnet, dass** sie zur Verabreichung in zwei Dosen bestimmt ist.

9. Impfstoffzusammensetzung nach einem der Ansprüche 5 bis 7, **dadurch gekennzeichnet, dass** sie zur Verabreichung durch subkutane Injektion in zwei Einzeldosen bestimmt ist, wobei die beiden Einzeldosen in einem zeitlichen Abstand von mindestens einem Monat verabreicht werden.

10. Verwendung von mindestens einem der Stämme nach einem der Ansprüche 1 bis 4 zur Herstellung einer Impfstoffzusammensetzung gegen das Dengue-Fieber.
